# EUROPEAN PATENT SPECIFICATION

(11) **EP 0 775 099 B1**
(45) Date of publication and mention of the grant of the patent: **20.10.1999**
(21) Application number: 96922191.0
(22) Date of filing: 11.06.1996
(51) Int. Cl.: C07C 2/20, B01J 27/32

(54) **BF3 PROMOTER PROCESS**
BF3-PROMOTER-VERFAHREN
PROCEDE PROMOTEUR DU BF3

(30) Priority: 13.06.1995 US 489858; 13.06.1995 US 489975
(43) Date of publication of application: 28.05.1997
(73) Proprietor: AMOCO CORPORATION, Chicago, IL 60601-0703 (US)
(72) Inventor: HOLUB, Richard, A., Houston, TX 77059 (US); SOLTIS, Scott, D., Pasadena, Texas, TX 77505 (US); HERMANN, Cynthia, W., Pearland, TX 77584 (US)
(74) Representative: Ritter, Stephen David
(86) International application number: IB9600735
(87) International publication number: WO9641782

(56) References cited:
- EP-A- 0 318 186
- EP-A- 0 364 889
- EP-A- 0 594 065
- US-A- 4 263 467

## Description

The invention relates to the improved utilization or recovery of boron trifluoride (BF₃) in a process for the oligomerization of an olefin or olefin-containing mixture wherein BF₃ is employed as a catalyst or catalyst component. In particular, the invention concerns the recovery of boron trifluoride (BF₃) from crude reaction product mixture derived from the oligomerization of an olefin or olefin-containing mixture.

### Background Of The Invention

The production of oligomers from olefin or olefin-containing mixtures, particularly from alpha olefins, is well known, as is the use of the oligomerization product or products in a variety of lubricants and functional fluids. In preferred processes for the oligomerization of olefins, boron trifluoride is employed as a catalyst for the oligomerization reaction. However, because BF₃ is a relatively slow catalyst, a "promoter" or co-catalyst composition, which may be selected from a wide variety of materials, is commonly employed with the BF₃ to improve reaction rates.

In at least one process utilizing BF₃ and a promoter, the promoter composition, e.g., an oxygenated organic material, such as an alkanol, is believed to form a stable complex or adduct with BF₃ supplied to the reaction zone. Preferably, the BF₃ is supplied to the reaction zone in an excess of that required for formation of the complex. In the case of such oxygen-containing promoter, for example, the BF₃ is commonly supplied in a ratio of from about 1.0 moles to about 4.0 moles of BF₃ per mole of oxygen atoms in the compound, although there is no actual limit. The excess BF₃ is believed to be loosely associated with the complex, or may simply be dissolved in the reaction mixture, and is susceptible to the recovery procedure of the invention.

EP-A- 0 364 889 discloses a process for the manufacture of an olefin oligomer, comprising the steps of polymerizing an olefin monomer in the presence of boron trifluoride and a boron trifluoride-alcohol complex as catalysts. to prepare a first oligomerization product, and then carrying out one of following steps (a) to (c): (a) removing boron trifluoride from the above first product by (i) placing the product under a reduced pressure, (ii) blowing an inert gas into the product, or (iii) heating the product at a relatively low temperature, to thereby prepare a second oligomerization product, and then subjecting the second product to a precipitation treatment to separate the boron trifluoride-alcohol complex therefrom, (b) heating the above first product at a relatively elevated temperature to recover boron trifluoride, or (c) centrifuging the above first product to recover the boron trifluoride-alcohol complex. These steps allow the catalysts to be recovered while maintaining the activity thereof at a desired level.

Prior to the invention, in at least one olefin oligomerization process utilizing BF₃ and promoter, the BF₃ catalyst remaining in the oligomerization reaction product mixture, upon recovery of the mixture, has represented a significant cost/disposal problem. That is, BF₃ recovery in a reusable form from the reaction product mixture has been difficult because of the unusual properties of BF₃ and the nature of oligomerization chemistry. As a result, rather than attempt recovery of the BF₃, the catalyst has been treated in a variety of ways, such as by reaction thereof with an ammonium or alkali metal compound, followed by appropriate disposal of the BF₃ reaction product. However, increasing environmental consciousness has spurred efforts to develop methods to reclaim such byproducts and thereby minimize environmental impact. Additionally, the loss of this valuable catalytic material by disposal directly impacts the economics of the oligomerization process. Accordingly, a need has existed for recovering BF₃, values from oligomer reaction systems or mixtures. The invention addresses this need.

### Summary Of The Invention

In one embodiment, therefore, the invention relates to a process in which a crude polyolefin-containing oligomerization reaction product liquid or liquid mixture containing dissolved BF₃ is recovered, and the dissolved BF₃ in the crude liquid mixture is vaporized therefrom in a vaporization zone. As used herein, the term "crude oligomerization reaction product mixture" is understood to refer to a liquid polyolefin-containing product mixture derived or obtained from a reaction zone or zones wherein an olefin is oligomerized in the presence of BF₃, as described more fully hereinafter. Additionally, the term "dissolved BF₃" is understood to refer not only to BF₃ that is dissolved in the reaction mixture but to include that BF₃ which may be loosely chemically held in the mixture. In this regard, evidence exists that BF₃ exists in the reaction mixture in different degrees of association. While not wishing to be bound by any theory of invention, it appears that BF₃ forms, on an equimolar basis, a strongly bonded complex with components of various promoter materials, e.g., the oxygen in oxygen-containing materials. This complex is not readily removed by vaporization from the reaction mixture. Additional BF₃ in or added to the mixture appears to be dissolved therein or only lightly bonded to the promoter or component thereof, and is susceptible to the recovery procedure of the invention.

The vaporized BF₃ leaves or is removed from the vaporization zone, and is contacted in an absorption zone with a specified olefin composition, or with an olefin composition and a promoter composition which is capable of forming a complex with BF₃, under conditions suitable for absorbing BF₃ or BF₃-complex in the olefin composition and, when the promoter composition is used, for forming such complex.

In general, when the BF₃ is absorbed using olefin without promoter, the olefin composition is supplied at a temperature sufficiently low, or the temperature in the absorption zone is maintained sufficiently low, so that, in combination with the quantity of liquid olefin composition supplied, BF₃ from the vaporization zone dissolves in the olefin composition to form dissolved BF₃. Most desirably, the volume of olefin composition supplied and the temperature thereof or of the absorption zone are so controlled that all of the BF₃ supplied to the absorption zone, on a continuous basis, is absorbed in the liquid olefin composition.

When BF₃ from the vaporization zone is contacted in the absorber with olefin and promoter, preferably, the quantities of BF₃, olefin composition, and promoter composition are regulated to form BF₃-promoter complex with little additional BF₃ dissolving in the olefin composition to form dissolved BF₃; an excess of BF₃, i.e., a ratio of greater than about 1:1, which would form dissolved BF₃, is preferably avoided.

In both embodiments of the invention, the mixture, comprising olefin composition, BF₃-promoter complex, and/or dissolved BF₃, may then be forwarded or transferred to a reaction zone or zones, where, under appropriate conditions, oligomerization of the olefin is permitted or allowed to occur by addition of BF₃ or BF₃ and promoter. In a preferred embodiment, the invention also relates to a process for the oligomerization of an olefin in the presence of BF₃ catalyst and promoter, the process being characterized by removal of crude reaction product mixture and treatment as described to recover dissolved BF₃.

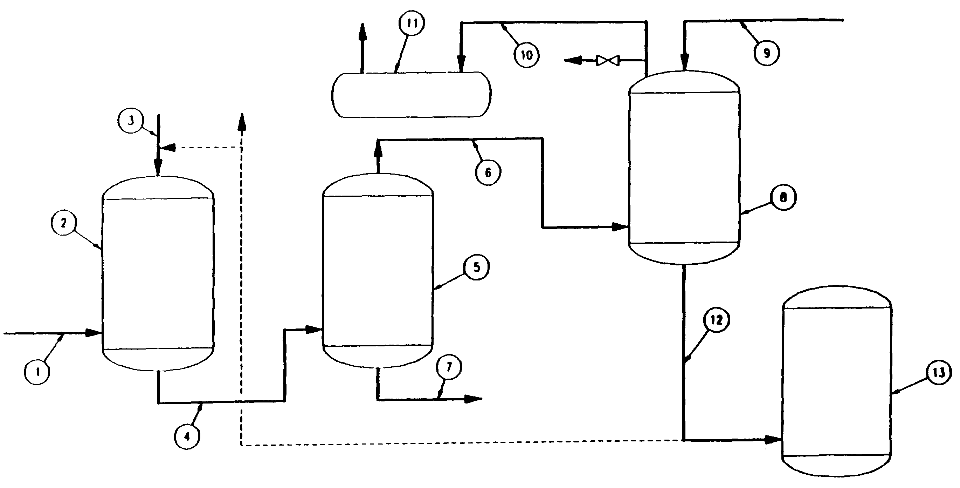

### Brief Description Of The Drawing

The drawing is a schematic illustration of the process flow type.

### Detailed Description Of The Invention

Processes for the oligomerization of an olefin or olefins utilizing BF₃ and a promoter are well known, as exemplified in U.S. patent 4,981,578, U.S. patent 4,032,591, and U.S. patent 4,409,415. Accordingly, only sufficient description of the reaction procedure is provided as is necessary for an understanding of the invention. Olefins commonly oligomerized by such procedures include linear and branched C₄ to C₃₀ olefins, preferably C₆ to C₂₀, which may be alpha or internal olefins, and mixtures thereof. Alpha olefins, and mixtures thereof, are preferred, particularly those containing 8 through 12 carbon atoms. The most preferred is 1-decene, usually supplied as a 1-decene composition which may be the pure or substantially pure material or a mixture comprising a substantial portion, say at least fifty percent by weight of 1-decene, such as a 75 weight percent mixture of 1-decene and other olefins. For simplicity, as used hereinafter, the term "olefin composition" is understood to include an olefin, a mixture of olefins, or an olefin- or olefins- containing composition, of the types above-mentioned, encompassing the presence, even in major amounts, of non-interfering or at least substantially non-interfering species, e.g., alkanes, in the composition. The term "1-decene composition", in a similar vein, is taken to include compositions ranging from "pure" 1-decene to mixtures with other olefins, or mixtures thereof, and includes, as noted, the presence, even in major amounts, of non-interfering or at least substantially non-interfering species, in the composition. Again, unless otherwise indicated or inconsistent with the circumstances, all percentages of components of a mixture expressed herein are by weight, based on the total weight of the mixture.

In general, the oligomerization reaction is carried out by combining the olefin composition and BF₃, preferably with the BF₃-promoter complex, in a reaction zone under suitable reaction conditions. Variation of reaction conditions is known to affect the character of the reaction product obtained; an oligomer of desired composition and properties may be obtained, for example, by regulating the temperature of the reaction. In this regard, the reaction will be conducted at suitable temperatures, e.g., from about -20°C to about 90°C, with temperatures within the range of from about 20°C to about 90°C being preferred. Similarly, pressures in the reactor may be varied, but normally will range from about one atmosphere to about 10 atmospheres (101.3 - 1013 kPa), with pressures of from about 1.5 atmospheres to about 5.0 atmospheres (152 - 506 kPa) being preferred.

Any of the known promoters that form a complex with BF₃ may be used. For example, straight and branched alkanols of 1 through 20 carbon atoms, (such as methanol, ethanol, n-propanol, isobutanol, n-hexanol, 2-ethylhexanol, n-decanol, n-dodecanol, and the like), and mixtures thereof, may be used. Also, water, fatty acids, i.e., hydrocarbyl acids containing from 1 to 20 carbon atoms (such as valeric, caproic, and the like), and mixtures thereof, organic esters (such as butyl acetate, methyl valerate, ethyl octanoate, and the like) and mixtures thereof, ketones (e.g., methyl ethyl ketone, methyl isobutyl ketone, and the like) and mixtures thereof, ethers (e.g., dibutyl ether, tetrahydrofuran, dioxane, and the like) and mixtures thereof, alkoxylated alcohols (such as 2-methoxyethanol, 2-ethoxyethanol, and the like) and mixtures thereof, polyhydric alcohols (e.g., glycol, glycerol, and the like) and mixtures thereof, inorganic acids (e.g., phosphoric and the like) and mixtures of such acids, silica, zeolites, and the like may be used. Preferred promoters are straight and branched chain alkanols containing from 1 through 8 carbons, with straight and branched chain alkanols containing from 2 through 5 carbons being most preferred. The promoter-complex and/or BF₃, is present in a catalytic amount, as understood by those skilled in the art.

As mentioned, dissolved BF₃ in the crude reaction product mixture removed from the oligomerization reaction zone is vaporized from the crude reaction product mixture. The vaporization may be accomplished by any suitable combination of temperature and pressure. The temperature utilized will be a temperature sufficient to vaporize dissolved BF₃ from the crude oligomerization reaction product mixture, but insufficient to decompose or dissociate BF₃. If a promoter or co-catalyst complex is present in the mixture, the vaporization temperature will be insufficient to dissociate or decompose the BF₃-promoter complex or co-catalyst complex. As used herein, the expressions "insufficient to decompose" and "insufficient to dissociate", in the context of the vaporization procedure of the invention and with respect to the BF₃-promoter complex, are understood to contemplate or allow minor or very minor decomposition or dissociation of the BF₃-promoter complex or co-catalyst, provided at least the bulk of the complex or co-catalyst remains undissociated or combined. Preferably, vaporization is accomplished by heating the liquid oligomerization reaction product mixture to a suitable temperature and allowing the BF₃ to flash therefrom at atmospheric or reduced pressure. In general, temperatures of from about 10°C to about 80°C will release the BF₃ at atmospheric or below atmospheric pressure, with temperatures within the range of from about 20°C to about 80°C being preferred at the pressures mentioned. The relatively low temperatures for 5 vaporization have the great advantage of minimizing undesirable effects on the oligomer in the crude reaction product mixture. Additionally, as a practical matter, since the BF₃ vaporized is to be reabsorbed or captured, lower temperatures in the range are desirable. The vaporization may be carried out in a zone or vessel which is "dedicated" to a specific oligomerization reactor or reaction zone, or, if multiple reactors or reaction zones are employed, a common vaporization vessel or zone may be used to vaporize the BF₃ from reaction mixture from multiple reaction zones.

Upon vaporization, the BF₃ passes or is forwarded to an absorption or contacting zone where it is contacted with a liquid olefin composition and a promoter of the types mentioned. Single or multiple absorption or contacting vessels may be employed to receive the vaporized BF₃ from one or more vaporization vessels or zones, or the absorption "zone" may comprise one or more vessels with blending and/or concurrent introduction of the BF₃. The type of vessel or vessels employed in the contacting is not critical; the contacting may be carried out, e.g., in a countercurrent spray contactor or contactors, tray tower(s), or packed column(s). Preferably, the promoter and the liquid olefin composition are blended and fed together, but it is possible to feed them separately to the absorption zone if intimate mixing is employed and conditions are otherwise appropriate for complex or adduct formation.

When using olefin without promoter for the absorption, BF3 is absorbed or dissolved in the absorption zone under suitable conditions of temperature and pressure into the liquid olefin composition. Preferably, the volumes or flow of the olefin composition are controlled or regulated, as indicated, so that all the BF₃ forwarded from the vaporization zone will be absorbed or dissolved in the olefin composition. The temperatures in the absorber should be maintained or regulated at levels that allow effective absorption of all BF₃ fed thereto; this may be accomplished preferably by chilling the olefin composition before entry into the absorption zone and/or by providing effective cooling in the absorption zone. Preferably, the temperature of liquid olefin composition on entry will be below about 40°C, most preferably 30°C, and the temperature in the absorber should not be allowed to exceed these values. Generally, the temperature will range from -20°C to 40°C, preferably 10°C to 30°C.

When using olefin and promoter in the absorption zone, BF₃ is absorbed or dissolved into the olefin composition or reacts directly with the promoter, and BF₃-promoter complex is formed, the complex being in or dissolving in the liquid olefin composition. Reaction conditions may be varied considerably. In general, such conditions will include a temperature below that at which the bulk of the complex to be formed will dissociate, e.g., in the case of a BF₃-alkanol complex, below about 110°C, and, with respect to pressures, at pressures of from below atmospheric to 20 atmospheres (2026 kPa) or greater, preferably 0.5 to 10 atmospheres (152 - 1013 kPa). Preferably, the volumes or flows of liquid olefin composition and promoter, or promoter-containing olefin composition are controlled or regulated, as indicated, to maintain as nearly as possible the desired ratio of the BF₃ to the promoter in the mixture formed. That is, the flows or volumes of olefin composition and promoter or promoter-containing olefin are regulated so that a significant excess of BF₃ does not occur in the olefin-BF₃-promoter mixture formed. Preferably, the ratio of BF₃ to promoter does not exceed a 1 to 1 ratio, in the case of oxygen compounds, with respect to the number of oxygen atoms in the promoter. As will be understood by those skilled in the art, excess of BF₃ will simply be absorbed as dissolved BF₃.

In view of the ready formation of the complex and the possible dissolution of excess BF₃, temperatures in the absorber preferably should be maintained or regulated so that oligomerization does not occur to any extent; this may be accomplished by chilling the liquid olefin composition before entry into the absorption zone and/or by providing effective cooling in the absorption zone. Preferably, the temperature of the liquid olefin composition on entry will be below about 40°C, most preferably 30°C, and the temperature in the absorber should not be allowed to exceed these values. Generally, the temperatures will range from -20°C to 40°C, preferably 10°C to 30°C.

In order to describe the invention more fully, the following illustrations are made utilizing the accompanying drawing. In these illustrations, all values given are calculated or merely exemplary, and the procedure is assumed to be continuous.

In a first illustration, vaporized BF₃ is absorbed in liquid olefin. As shown, an olefin-containing stream, e.g., a stream which may be 1-decene 5 (75 percent 1-decene monomer, 25 percent other olefins and inert material), which further contains 0.14 percent propanol in line 1 enters reactor 2 where it is contacted with BF₃ supplied via line 3. Reactor 2, although illustrated as a single vessel, is preferably a series of vessels with feeds of BF₃ to each vessel, reacting liquid passing from one vessel to the next in the series, with o crude oligomerization reaction product mixture being removed from the last vessel. The contacting is carried out under conditions to oligomerize the olefin, accompanied by initmate mixing of the reactants, total contact time being, for example, about two hours and ten minutes. A crude polydecene oligomerization reaction product mixture containing dissolved BF₃ is removed 5 from the bottom of reactor 2 and forwarded via line 4 to a flash or vaporization zone 5. Flash zone 5 comprises a simple tank where the BF₃ is flashed from the crude reaction mixture by suitable temperature-pressure conditions. For example, prior to entry into zone or vessel 5, the crude oligomerization reaction product mixture may be heated to a temperature of o .70°C, so that upon entry into tank 5, which is operated at slightly below atmospheric pressure, the BF₃ in the mixture flashes from the crude oligomerization reaction product mixture and passes overhead through line 6. The remaining crude oligomerization reaction product mixture, containing BF₃-complex, is removed from flash zone 5 via line 7 to further treatment and recovery of the contents thereof. BF₃ in line 6 is forwarded to absorption zone 8 where it is contacted intimately with a liquid olefin stream from line 9 comprising 1-decene (75 percent 1-decene monomer, 25 percent other olefins and inert material). Vessel 8 preferably comprises a packed tower wherein the BF₃ is passed countercurrent to the liquid olefin, inert material entering with the BF₃ passing overhead via line 10 to vent scrubber 11. The liquid olefin stream in line 9 is preferably at or cooled to a temperature of about 10°C to assist in the absorption of BF₃; alternately or additionally the vessel may be cooled by suitable heat exchange means to ensure that effective dissolution of the BF₃ occurs while possible oligomerization of the olefin in the absorber is minimized. Accordingly, there is formed in absorber 8 a liquid olefin mixture containing, for example, 0.16 percent dissolved BF₃. This mixture, containing the vaporized BF₃ values from vessel 5, is admirably suited for oligomerization, and is forwarded via line 12 to reactor 13 wherein additional BF₃ and promoter, such as propanol, are added to raise the concentration of BF₃, and increase the ratio of BF₃ above 1 to 1 with respect to the propanol. Reactor 13 is maintained at appropriate reaction conditions, such as about 100°F (38°C) and 44 psig (303 kPa). Alternately (dotted line), the mixture in 12 may be returned to reaction zone 2 for reaction therein, or to some other site for utilization. Although not illustrated, the crude reaction mixture from 13 may be treated in a similar fashion to that from reactor 2, to the end that economic utilization of BF₃ is obtained.

In a second illustration, vaporized BF₃ is contacted with olefin and promoter in an absorption zone to form a liquid olefin mixture containing BF₃-promoter complex. As shown, an olefin-containing stream, e.g., a stream of 1-decene (75 percent 1-decene monomer, 25 percent other olefins and inert material), which may be a recycle stream, and which further contains 0.58 percent propanol, or 1.23 percent propanol adduct, in line 1 enters reactor 2 where it is contacted with BF₃ supplied via line 3. Reactor 2, although illustrated as a single vessel, is preferably a series of vessels with feeds of BF₃ to each vessel, reacting liquid passing from one vessel to the next in the series, with crude oligomerization reaction product mixture being removed from the last vessel. The contacting is carried out under conditions to oligomerize the olefin, e.g., 78°C and 70 psig (482 kPa) pressure, accompanied by intimate mixing of the reactants, total contact time being, for example, 5.5 hours. A crude polydecene oligomerization reaction product mixture containing dissolved BF₃ is removed from the bottom of reactor 2 and forwarded via line 4 to a flash or vaporization zone 5. Flash zone 5 comprises a simple tank where the BF₃ is flashed from the crude reaction mixture by suitable temperature-pressure conditions. For example, prior to entry into zone or vessel 5, the crude polydecene oligomerization reaction product mixture may be heated to a temperature of 70°C, so that upon entry into tank 5, which is operated at slightly below atmospheric pressure, the BF₃ in the mixture flashes from the crude oligomerization reaction product mixture and passes overhead through line 6. The remaining crude oligomerization reaction product mixture, containing BF₃-complex, is removed from flash zone 5 via line 7 to further treatment and recovery of the contents thereof. BF₃ in line 6 is forwarded to absorption zone 8 where it is contacted intimately with an olefin stream from line 9 containing a promoter composition, e.g., 1-decene (75 percent 1-decene monomer, 25 percent other olefins and inert material) and 0.58 percent propanol. Vessel 8 preferably comprises a sparging tower wherein the BF₃ is passed countercurrent to the sparging liquid olefin-promoter mixture, inert material entering with the BF₃ passing overhead via line 10 to vent scrubber 11. The liquid olefin stream in line 9 is preferably at or cooled to a temperature of about 10°C to assist in the absorption of BF₃, and in avoiding premature reaction of the olefin; alternately, or additionally, the vessel may be cooled by suitable heat exchange means to ensure that effective complexing of the BF₃ occurs while possible oligomerization of the olefin in the absorber is minimized. Accordingly, there is formed in absorber 8 a liquid olefin mixture containing BF₃-promoter complex and possibly dissolved BF₃. This mixture, which has captured the vaporized BF₃ values from vessel 5, is admirably suited for oligomerization, and is forwarded via line 12 to reactor 13 wherein additional BF₃ is added and appropriate reaction conditions are maintained. Alternately (dotted line), the mixture from 12 may be returned to vessel 2 for reaction therein, or to some other site for utilization. Although also not illustrated, the crude reaction mixture from 13 may be treated in a similar fashion to that from reactor 2, to the end that economic utilization of BF₃ is obtained.

The advantage of close temperature control of the absorbing liquid olefin composition, i.e., either providing a cooled liquid olefin composition or ensuring effective cooling in the absorber, is supported by the following experiments.

Examples I and II illustrate absorption of BF₃ with olefin; Examples III and IV illustrate use of olefin and promoter for absorption.

### Example I

Dry 1-decene was added to an autoclave, and BF₃ was added under minimal pressure (4 inches (101 mm) of water) until saturation was achieved, the temperature of the 1-decene being held at 30°C. The concentration of BF₃. absorbed at this temperature was determined by quantitative technique involving the hydrolysis of the saturated mixture and aqueous phase metals analysis for boron content by an inductively coupled plasma analytical instrument. The analysis showed the saturation concentration of BF₃ at 30°C to be 0.19 wt. percent.

### Example II

The procedure of Example I was repeated, except that the temperature was maintained at 10°C. Analysis showed the saturation concentration of BF₃ at 10°C to be 0.28 wt. percent, an increase of over 47 percent by weight.

### Example III

Two hundred twenty-five grams of a 4.3 percent solution of n-propanol-BF₃ adduct was introduced into a reactor, the mixing of the reactants being accomplished with high agitation. The temperature in the reactor reached 30°C, and the reaction was conducted for ten minutes. Analysis of the reaction mixture showed the degree of oligomerization for the 1-decene to be 8.5 percent, and the degree of isomerization to be 1 percent.

### Example IV

The procedure of Example III was repeated, except that the temperature of the reactor was held at 10°C. Analysis of the reaction mixture showed the degree of oligomerization to be only 4.5 percent, and the degree of isomerization to be a mere 0.05 percent.

While the invention has been illustrated with particular apparatus, those skilled in the art will appreciate that, except where specified or otherwise required, other equivalent or analogous units may be employed. As indicated, the terms "zone" or "zones", as employed in the specification and claims, include, where suitable, the use of segmented equipment operated in series, or the division of one unit into multiple units because of size constraints, etc. For example, an absorption column might comprise two separate columns or vessels in which the liquid from the lower portion of the first column would be introduced into the upper portion of the second column, the gaseous material from the upper portion of the first column being fed to the lower portion of the second column.

## Claims

1. A process for the recovery of BF₃ comprising recovering crude oligomerization reaction product mixture containing dissolved BF₃;
vaporizing BF₃ from said crude oligomerization reaction product mixture in a vaporization zone at a temperature insufficient to decompose BF₃ or any BF₃-promoter complex in said mixture;
contacting BF₃ from said vaporization zone with a liquid olefin composition and, optionally at least one promoter composition capable of forming a complex with BF₃, in an absorption zone, under conditions to absorb BF₃ in said liquid olefin composition and, when the promoter composition is present, to form a BF₃-promoter complex, producing a mixture comprising liquid olefin composition and BF₃ or BF₃-promoter complex or a combination thereof.

2. The process of claim 1 wherein the mixture comprising liquid olefin composition and BF₃ or BF₃-promoter complex is contacted in a reaction zone with BF₃ under conditions to oligomerize said liquid olefin.

3. A process for the oligomerization of an olefin composition and recovery of BF₃ comprising contacting an olefin composition in a reaction zone with BF₃ under conditions to oligomerize said olefin, forming a crude polyolefin oligomerization reaction product mixture containing dissolved BF₃;
recovering crude oligomerization reaction product mixture containing dissolved BF₃;
vaporizing BF₃ from said crude oligomerization reaction product mixture in a vaporization zone at a temperature insufficient to decompose BF₃ or any BF₃ promoter complex in said mixture;
contacting BF₃ from said vaporization zone with a liquid olefin composition and a promoter composition capable of forming a complex with BF₃, in an absorption zone, under conditions to form a BF₃-promoter complex, producing a mixture comprising liquid olefin composition and BF₃-promoter complex.

4. A process for the oligomerization of an olefin composition and recovery of BF₃ comprising contacting an olefin composition in a reaction zone with BF₃ under conditions to oligomerize said olefin, forming a crude polyolefin oligomerization reaction product mixture containing dissolved BF₃;
recovering crude oligomerization reaction product mixture containing dissolved BF₃;
vaporizing BF₃ from said crude oligomerization reaction product mixture in a vaporization zone at a temperature insufficient to decompose BF₃ or any BF₃ promoter complex in said mixture;
contacting BF₃ from said vaporization zone with a liquid olefin composition in an absorption zone under conditions to absorb BF₃, in said liquid olefin composition, forming a mixture comprising liquid olefin composition and BF₃

5. The process of any of claims 14 wherein the olefin composition is selected from linear and branched olefins containing from 6 to 20 carbon atoms.

6. The process of any of claims 1-5 wherein said BF₃-promoter complex is the reaction product of BF₃, and a composition selected from straight and branched alkanols of 1 through 20 carbon atoms, and mixtures thereof, water, hydrocarbyl acids containing from 1 to 20 carbon atoms, and mixtures thereof, organic esters, and mixtures thereof, ketones, and mixtures thereof, ethers, and mixtures thereof, alkoxylated alkanols, and mixtures thereof, polyhydric alcohols, and mixtures thereof, inorganic acids, and mixtures thereof, silica, zeolites, and mixtures thereof; and mixtures thereof.

7. The process of any of claims 1-6 wherein said BF₃-promoter complex is the reaction product of BF₃ and a composition selected from straight and branched alkanols of 1 through 20 carbon atoms, and mixtures thereof.

8. The process of any of claims 1-3 and 5-7 wherein, in the absorption zone, the ratio of BF₃ from the vaporization zone to promoter composition is about 1:1 or less.

9. The process of any of claims 1-8 wherein the liquid olefin composition comprises 1-decene.

## Patentansprüche

1. Verfahren zur Wiedergewinnung von BF₃, welches umfaßt, daß ein rohes Produktgemisch der Oligomerisierungsreaktion, welches gelöstes BF₃ enthält, wiedergewonnen wird; daß BF₃ aus diesem rohen Produktgemisch der Oligomerisierungsreaktion in einer Verdampfungszone bei einer Temperatur, die nicht zur Zersetzung von BF₃ oder eines BF₃/Promotor-Komplexes in diesem Gemisch ausreicht, verdampft wird; und daß das BF₃ aus der Verdampfungszone mit einer flüssigen Olefin-Zusammensetzung und gegebenenfalls mindestens einer Promotor-Zusammensetzung, welche dazu befähigt ist, einen Komplex mit BF₃ zu bilden, in einer Absorptionszone unter Bedingungen in Berührung gebracht wird, bei denen BF₃ in der flüssigen Olefin-Zusammensetzung absorbiert wird und, sofern eine Promotor-Zusammensetzung anwesend ist, ein BF₃/Promotor-Komplex gebildet wird, wodurch eine Mischung hergestellt wird, welche eine flüssige Olefin-Zusammensetzung und BF₃ oder einen BF₃/Promotor-Komplex oder eine Kombination davon umfaßt.

2. Verfahren nach Anspruch 1, worin die Mischung, welche eine flüssige Olefin-Zusammensetzung und BF₃ oder einen BF₃/Promotor-Komplex umfaßt, in einer Reaktionszone unter Bedingungen mit BF₃ in Berührung gebracht wird, bei denen das flüssige Olefin oligomerisiert wird.

3. Verfahren zur Oigomerisierung einer Olefin-Zusammensetzung und zur Wiedergewinnung von BF₃, welches umfaßt, daß eine Olefin-Zusammensetzung in einer Reaktionszone mit BF₃ unter Bedingungen in Berührung gebracht wird, bei denen das Olefin oligomerisiert, wobei ein rohes Produktgemisch der Polyolefin-Oligomerisierungsreaktion gebildet wird, welches gelöstes BF₃ enthält; daß das rohe Produktgemisch der Oligomerisierungsreaktion, welches gelöstes BF₃ enthält, wiedergewonnen wird; daß BF₃ aus dem rohen Produktgemisch der Oligomerisierungsreaktion in einer Verdampfungszone bei einer Temperatur verdampft wird, die nicht zur Zersetzung von BF₃ oder eines BF₃/Promotor-Komplexes in diesem Gemisch ausreicht; und daß das BF₃ aus der Verdampfungszone mit einer flüssigen Olefin-Zusammensetzung und einer Promotor-Zusammensetzung, welche dazu befähigt ist, einen Komplex mit BF₃ zu bilden, in einer Absorptionszone unter Bedingungen in Berührung gebracht wird, bei denen ein BF₃/Promotor-Komplex gebildet wird, wodurch eine Mischung hergestellt wird, welche eine flüssige Olefin-Zusammensetzung und einen BF₃/Promotor-Komplex umfaßt.

4. Verfahren zur Oligomerisierung einer Olefin-Zusammensetzung und zur Wiedergewinnung von BF_{3,} welches umfaßt, daß eine Olefin-Zusammensetzung in einer Reaktionszone mit BF₃ unter Bedingungen in Berührung gebracht wird, bei denen das Olefin oligomerisiert, wodurch ein rohes Produktgemisch der Polyolefin-Oligomerisierungsreaktion gebildet wird, welches gelöstes BF₃ enthält; daß das rohe Produktgemisch der Oligomerisierungsreaktion, welches gelöstes BF₃ enthält, wiedergewonnen wird; daß BF₃ aus dem rohen Produktgemisch der Oligomerisierungsreaktion in einer Verdampfungszone bei einer Temperatur verdampft wird, die nicht zur Zersetzung von BF₃ oder eines BF₃/Promotor-Komplexes in diesem Gemisch ausreicht; und daß das BF₃ aus der Verdampfungszone mit einer flüssigen Olefin-Zusammensetzung in einer Absorptionszone unter Bedingungen in Berührung gebracht wird, bei denen BF₃ in der flüssigen Olefin-Zusammensetzung absorbiert wird, wodurch eine Mischung gebildet wird, welche eine flüssige Olefin-Zusammensetzung und BF₃ umfaßt.

5. Verfahren nach irgendeinem der Ansprüche 1 bis 4, worin die Olefin-Zusammensetzung ausgewählt ist aus linearen und verzweigten Olefinen mit 6 bis 20 Kohlenstoffatomen.

6. Verfahren nach irgendeinem der Ansprüche 1 bis 5, worin der BF₃/Promotor-Komplex das Reaktionsprodukt von BF₃ und einer Zusammensetzung ist, die ausgewählt ist aus geradkettigen und verzweigten Alkanolen mit 1 bis 20 Kohlenstoffatomen sowie Mischungen daraus, Wasser, Kohlenwasserstoff-Säuren mit 1 bis 20 Kohlenstoffatomen sowie Mischungen daraus, organischen Estern sowie Mischungen daraus, Ketonen sowie Mischungen daraus, Ethern sowie Mischungen daraus, alkoxylierten Alkanolen sowie Mischungen daraus, mehrwertigen Alkoholen sowie Mischungen daraus, anorganischen Säuren sowie Mischungen daraus, Siliciumdioxid, Zeolithen sowie Mischungen daraus; sowie Mischungen daraus.

7. Verfahren nach irgendeinem der Ansprüche 1 bis 6, worin der BF₃/Promotor-Komplex das Reaktionsprodukt von BF₃ und einer Zusammensetzung ist, die ausgewählt ist aus geradkettigen und verzweigten Alkanolen mit 1 bis 20 Kohlenstoffatomen sowie Mischungen daraus.

8. Verfahren nach irgendeinem der Ansprüche 1 bis 3 und 5 bis 7, worin das Verhältnis von BF₃ aus der Verdampfungszone zur Promotor-Zusammensetzung in der Absorptionszone etwa 1:1 oder weniger beträgt.

9. Verfahren nach irgendeinem der Ansprüche 1 bis 8, worin die flüssigen Olefin-Zusammensetzung 1-Decen umfaßt.

## Revendications

1. Procédé de récupération du BF₃ comprenant :
- la récupération du mélange brut des produits de réaction d'oligomérisation et contenant du BF₃ dissous ;
- la vaporisation du BF₃ à partir dudit mélange brut de produits de réaction d'oligomérisation dans une zone de vaporisation à une température insuffisante pour décomposer le BF₃ ou un quelconque complexe promoteur de BF₃ dans ledit mélange ;
- la mise en contact du BF₃ provenant de ladite zone de vaporisation avec une composition oléfinique liquide et éventuellement avec au moins une composition de promoteur capable de former un complexe avec BF₃ dans une zone d'absorption, dans des conditions permettant d'absorber BF₃ dans ladite composition oléfinique liquide, et lorsque la composition promoteur est présente, de former un complexe promoteur de BF₃, produisant ainsi un mélange comprenant la composition oléfinique liquide et le BF₃ ou le complexe promoteur de BF₃ ou une combinaison de ceux-ci.

2. Procédé selon la revendication 1, dans lequel le mélange comprenant la composition oléfinique et BF₃ ou le complexe promoteur de BF₃ est mis en contact dans une zone réactionnelle avec BF₃ dans des conditions d'oligomérisation de ladite oléfine liquide.

3. Procédé d'oligomérisation d'une composition oléfinique et de récupération de BF₃ comprenant :
- la mise en contact d'une composition oléfinique dans une zone réactionnelle avec BF₃ dans des conditions d'oligomérisation de ladite oléfine, formant ainsi un mélange brut de produits de réaction d'oligomérisation polyoléfinique contenant du BF₃ dissous ;
- la récupération du mélange brut de produits de réaction d'oligomérisation contenant du BF₃ dissous
- la vaporisation du BF₃ à partir dudit mélange brut de produits de réaction d'oligomérisation dans une zone de vaporisation, à une température insuffisante pour décomposer le BF₃ ou un quelconque complexe promoteur de BF₃ dans ledit mélange ;
- la mise en contact du BF₃ provenant de ladite zone de vaporisation avec une composition oléfinique liquide et une composition de promoteur capable de former un complexe avec BF₃ dans une zone d'absorption, dans des conditions de formation d'un complexe promoteur de BF₃, produisant ainsi un mélange comprenant la composition oléfinique liquide et le complexe promoteur de BF₃.

4. Procédé d'oligomérisation d'une composition oléfinique et de récupération de BF₃ comprenant :
- la mise en contact d'une composition oléfinique dans une zone réactionnelle avec BF₃ dans des conditions d'oligomérisation de ladite oléfine, formant ainsi un mélange brut de produits de réaction d'oligomérisation polyoléfinique contenant du BF₃ dissous ;
- la récupération du mélange brut de produits de réaction d'oligomérisation contenant du BF₃ dissous
- la vaporisation du BF₃ à partir dudit mélange brut de produits de réaction d'oligomérisation dans une zone de vaporisation, à une température insuffisante pour décomposer le BF₃ ou un quelconque complexe promoteur de BF₃ dans ledit mélange ;
- la mise en contact du BF₃ provenant de ladite zone de vaporisation avec une composition oléfinique liquide dans une zone d'absorption dans des conditions permettant d'absorber BF₃ dans ladite composition oléfinique, formant ainsi un mélange comprenant la composition oléfinique liquide et BF₃.

5. Procédé selon l'une quelconque des revendications 1 à 4, dans lequel la composition oléfinique est choisie parmi les oléfines linéaires et ramifiées contenant 6 à 20 atomes de carbone.

6. Procédé selon l'une quelconque des revendications 1 à 5, dans lequel ledit complexe promoteur de BF₃ est le produit de la réaction de BF₃ et d'une composition choisie parmi les alcanols linéaires et ramifiés contenant 1 à 20 atomes de carbone et leurs mélanges, l'eau, les acides hydrocarbyliques contenant 1 à 20 atomes de carbone et leurs mélanges, les esters organiques et leurs mélanges, les cétones et leurs mélanges, les éthers et leurs mélanges, les alcanols alcoxylés et leurs mélanges, les alcools polyhydriques et leurs mélanges, les acides minéraux et leurs mélanges, la silice, les zéolites et leurs mélanges et les mélanges de qes produits.

7. Procédé selon l'une quelconque des revendications 1 à 6, dans lequel ledit complexe promoteur de BF₃ est le produit de la réaction de BF₃ et d'une composition choisie parmi les alcanols linéaires et ramifiés contenant 1 à 20 atomes de carbone et leurs mélanges.

8. Procédé selon l'une quelconque des revendications 1 à 3 et 5 à 7, dans lequel, à l'intérieur de la zone d'absorption, le rapport entre le BF₃ provenant de la zone de vaporisation et la composition de promoteur est d'environ 1/1 ou moins.

9. Procédé selon l'une quelconque des revendications 1 à 8, dans lequel la composition oléfinique liquide comprend du 1-décène.
